# EUROPEAN PATENT SPECIFICATION

(11) **EP 0 324 578 B1**
(45) Date of publication and mention of the grant of the patent: **07.07.1993**
(21) Application number: 89300179.2
(22) Date of filing: 10.01.1989
(51) Int. Cl.: A61F 13/15

(54) **Disposable diaper with improved hook and loop fastener system**
Wegwerfwindel mit Haftverschluss
Couche à jeter à fermeture du type à crochets et à boucles

(30) Priority: 11.01.1988 US 142552
(43) Date of publication of application: 19.07.1989
(73) Proprietor: MINNESOTA MINING AND MANUFACTURING COMPANY, St. Paul, Minnesota 55133-3427 (US)
(72) Inventor: Zoia, Anthony J. c/o Minnesota Mining and, St. Paul Minnesota 55144-1000 (US); Midgley, Roland R. c/o Minnesota Mining and, St. Paul Minnesota 55144-1000 (US); Plaschko, Donald L. c/o Minnesota Mining and, St. Paul Minnesota 55144-1000 (US); Melbye, William L. c/o Minnesota Mining and, St. Paul Minnesota 55144-1000 (US); Wood, Leigh E. c/o Minnesota Mining and, St. Paul Minnesota 55144-1000 (US); Nestegard, Susan K. c/o Minnesota Mining and, St. Paul Minnesota 55144-1000 (US)
(74) Representative: Baillie, Iain Cameron

(56) References cited:
- EP-A- 0 235 014
- EP-A- 0 276 970
- EP-A- 0 321 232
- EP-A- 0 321 234
- DE-A- 2 504 210
- FR-A- 2 516 757
- US-A- 4 047 529

## Description

### Technical Field

The present invention concerns hook and loop fastener systems that are used on inexpensive or disposable garments such as diapers.

### Background of the Invention

Various fasteners have been used on inexpensive or disposable garments such as diapers, including lengths of pressure-sensitive adhesive coated tape, snaps, and hook and loop fasteners.

Of these, lengths of pressure-sensitive adhesive coated tape are presently most widely used as the fasteners for disposable diapers. The lengths of tape both afford fastening the diaper in place on an individual such as a baby, and additionally, after the diaper has been soiled and removed from the individual, provide means for securing the soiled diaper in a rolled or folded condition surrounding the soiled portion of the diaper to facilitate its disposal. The presence of relatively small amounts of contaminants such as talcum powder or baby oil either on the pressure-sensitive adhesive or on the portion of the garment to which the pressure-sensitive adhesive is to be adhered by the user can reduce the reliability of such fasteners, however.

The use of hook and loop fasteners on inexpensive or disposable garments such as diapers substantially overcomes this problem of reduced fastener reliability due to contaminants such as talcum powder or baby oil, however, many hook and loop fasteners are too expensive to be economically used on disposable diapers. Thus, inexpensive portions for hook and loop fasteners are being developed that can securely close the diaper and allow a limited number (e.g., 10) openings and closings of the fastener without seriously degrading it, and are sufficiently inexpensive that they can economically be used on a disposable diaper or similar garment. While such hook and loop fasteners can provide secure fastening to hold the diaper in place and allow opening and closing of the fastener to inspect the condition of the diaper, the hook portion of the fastener can have a tendency to make unintended engagement with portions of the diaper before it is engaged with the loop portion of the fastener. More importantly, the location of the hook and loop fastener portions that allows the diaper to be attached to an individual does not typically allow those fastener portions to secure the diaper in a rolled or folded condition surrounding a soiled portion thereof after the diaper is removed from an individual.

In EP-A-0321232 and EP-A-0321234 (known under Article 54(3) and (4)) there is provided a disposable garment or diaper of the type including a generally rectangular laminate and hook and loop fastener means for fastening together portions of the laminate to secure the diaper to an individual, such as a baby. The fastener means includes loop fastener portion means adjacent a first end and including a multiplicity of loops, and a pair of flexible elongate rectangular polymeric tab assemblies having first end portions attached to the laminate adjacent an opposite second end and having distal end portions unattached to the laminate, and hook fastener portion means having a plurality of projecting hook members adapted to make releasable engagement with the loops on the loop fastener means along the distal end portions of the tab assemblies. The tab assemblies include a layer of pressure-sensitive adhesive on their distal end portions adjacent the hook fastener portion means, which layers of pressure sensitive adhesive provide, after the diaper has been soiled and removed from the individual, means for securing the soiled diaper in a rolled or folded condition surrounding the soiled portion of the diaper to facilitate its disposal by engagement of the layer of pressure sensitive adhesive with the laminate. Such securement of a soiled diaper is also disclosed in US-A-4047529.

### Disclosure of the Invention

The present invention provides a hook and loop fastening system for a disposable garment such as a diaper that both affords the advantage of reliability despite contaminants such as talcum powder or baby oil on the diaper, and provides means, after the diaper is soiled and removed, for securing the diaper in a rolled or folded condition surrounding the soiled portion of the diaper to facilitate its disposal.

According to one aspect of this invention there is provided a disposable garment or diaper as claimed in present claim 1.

According to another aspect of this invention there is provided a disposable garment or diaper as claimed in present claim 2.

### Brief Description of Drawings

The present invention will be further described with reference to the accompanying drawing wherein like reference numerals refer to like parts in the several views, and wherein:
Figure 1 is a perspective view of a disposable diaper useful in understanding the present invention;
Figure 2 is an enlarged fragmentary sectional view taken approximately along line 2-2 of Figure 1 useful in understanding the present invention, and shows detail of a tab assembly incorporated in the diaper of Figure 1 that includes hook fastener portion means and a layer of pressure sensitive adhesive used in disposing of the diaper, which tab assembly is shown in a stored position;
Figure 3 is an enlarged fragmentary sectional view similar to Figure 2 except that the tab assembly is shown in a ready position;
Figure 4 is a schematic view illustrating a method of applying the tab assembly shown in Figures 1 through 3;
Figure 5 is an enlarged section view which shows detail of a first embodiment of a tab assembly of a diaper in accordance with a first aspect of the present invention and includes a hook fastener portion and layer of pressure sensitive adhesive used in disposing of the diaper, which tab assembly is shown in a ready position;
Figure 6 is an enlarged sectional view similar to Figure 5 but shown with the tab assembly in a dispose position;
Figure 7 is an enlarged sectional view which shows details of an alternate embodiment of a tab assembly of a diaper in accordance with the first aspect of the present invention and includes a hook fastener portion and layer of pressure sensitive adhesive used in disposing of the diaper, which tab assembly is shown in a ready position;
Figure 8 is an enlarged sectional view similar to Figure 7 but shown with the tab assembly in a dispose position; and
Figure 9 is an enlarged sectional view which shows detail of a second alternate embodiment of a tab assembly of a diaper in accordance with a second aspect of the present invention and includes a hook fastener portion and layer of pressure sensitive adhesive used in disposing of the diaper, which tab assembly is shown in a ready position.

### Description of the Preferred Embodiments

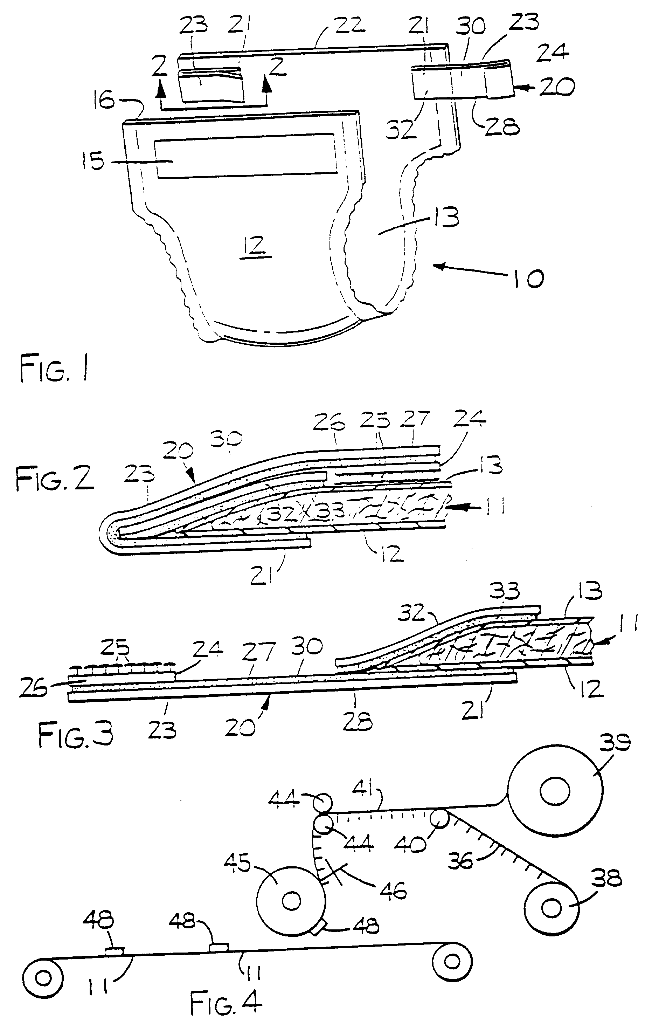

Referring now to Figure 1 there is shown a disposable garment or diaper generally designated by the reference numeral 10.

The diaper 10 is of the type including a generally rectangular laminate 11 including an outer liquid-impermeable polymeric film 12 and an inner absorbing layer 13. The diaper 10 also includes hook and loop fastener means for fastening together portions of the laminate 11 to secure the diaper 10 to an individual, such as a baby. The hook and loop fastening means includes loop fastener portion means, which, as illustrated is an elongate rectangular loop fastener portion 15 having a multiplicity of loops on its outer surface, which loop fastener portion 15 is adhered to the film 12 across what is intended to be the front of the diaper 10 adjacent and along a first end 16 of the rectangular diaper 10. Also included in the hook and loop fastening means are a pair of flexible elongate rectangular polymeric tab assemblies 20 having first end portions 21 attached to the laminate 11 adjacent an opposite second end 22 and having distal end portions 23 unattached to the laminate 11, and hook fastener means or portions 24 having a plurality of hook members 25 projecting from a backing 26 that are adapted to make releasable engagement with the loops on the loop fastener portion 15 with the backing 26 of each of the hook fastener portions 24 being attached to a different one of the tab assemblies 20 along its distal end portion 23. The tab assemblies 20 each include an elongate polymeric strip 28 having a layer 27 of pressure-sensitive adhesive entirely along on surface (i.e., a length of pressure sensitive adhesive coated tape of the type sold as KR-2272 by 3M Company, St. Paul, Minnesota). One end portion of the layer 27 of pressure sensitive adhesive helps attach the first end portion 21 of the tab assembly 20 to the laminate 11 and an opposite end portion adheres the backing 26 of the hook fastener portion 24 to the strip 28 along the distal end portion of the tab assembly 20. A central portion 30 of the layer 27 of pressure sensitive adhesive provides both means to position the tab assembly in a storage position, and, after the diaper 10 has been soiled and removed from the individual, means for securing the soiled diaper 10 in a rolled or folded condition surrounding the soiled portion of the diaper 10 (not shown) to facilitate its disposal by engagement of the layer 27 of pressure sensitive adhesive with the film 12 or other portions of the laminate 11.

Each of the tab assemblies 20 includes a release liner 32 having a major portion attached by a layer of adhesive 33 to the nonwoven layer 13 of the laminate 11 opposite the end portion of the strip 28 adhered to the film 12 and having a minor portion extending past the edge of the laminate 11 and adhered to the layer 27 of pressure sensitive adhesive. In a storage positions of the tab assemblies 20 used in shipping the diaper 10 to the user, their distal end portions 23 are folded over to releasably adhere the release liners 32 over the central portions 30 of the layers 27 of pressure sensitive adhesive on the distal end portions 23 adjacent the hook fastener portion 24 (see the left tab assembly 20 in Figure 1 and Figure 2) to protect the central portion 30 of the layer of pressure sensitive adhesive 27 and the hook fastener portions 24 from chance unintentional engagement with various substrates prior to application of the diaper 10. To use the diaper 10, a person may easily peel open the tab assemblies 20 to a ready position shown for the right tab assembly 20 in Figure 1 and in Figure 3 to afford releasable engagement of the hook fastener portions 24 with the elongate loop fastener portion 15. Subsequently, as indicated above, after the diaper 10 has been soiled and removed from the individual, the central portion 30 of at least one of the layers 27 of pressure sensitive adhesive provides means for securing the soiled diaper 10 in a rolled or folded condition surrounding the soiled portion of the diaper 10 (not shown) to facilitate its disposal by engagement of at least one of the central portions 30 of the layers 27 of pressure sensitive adhesive with the film 12 of the laminate 11.

The loop fastener portion 15 includes a backing layer which could be a nonwoven material, but is preferably a polymeric film (e.g., polyethylene), and has a plurality of through stitches formed with polymeric strands by a stitch-knitting machine such as the "Malimo" type Malipol Stitch-Knitting Machine manufactured by Textima in East Germany and distributed in the United States by Chima, Inc. of Reading, Pennsylvania, that form the multiplicity of loops 15 along its first surface adapted to be releasably mechanically engaged by the hooks on the mating hook fastener portions 24. Prior to being stitched to form the loops, the film backing layer may be printed with one or more symbols, including written or pictorial instructional material, a brand name, or a pattern or design to improve the aesthetic appeal of the diaper 10 or to serve as indices that aid the user in fitting diapers onto an infant consistently from fitting to fitting. Such printing remains functionally visible through the loops.

The unitary polymeric hook fastener portions 24 each comprise the plate like backing 26 that is thin strong and flexible, and a multiplicity of the resiliently flexible spaced hook members 25 projecting at generally a right angle from the upper surface of the backing 26. The hook members 25 each comprise a stem portion attached at one end to the backing 26, and a head portion at the end of the stem portion opposite the backing 26. The head portion projects past the stem portion on at least one of two opposite sides, and has a rounded surface opposite the stem portion to help the head portion enter between loops in the loop fastener portion 15. The hook members 25 are more easily and firmly engaged with many types of loop fastener portions than the hook members on known commercially available hook fastener portions, in large part because their head portions are very small in cross section compared to head portions on the hook members of those commercially available hook fastener portions, and thus more easily penetrate into a loop fastener portion. Specifically, the hook members 25 each have a height dimension from the upper surface of the backing 26 of less than 1.5 millimeter (0.06 inch) and preferably of about 0.10 centimeter (0.04 inch). The stem and head portions each have generally the same thickness dimension of less than 0.046 centimeter (0.018 inch) and preferably in the range of 0.020 to 0.028 centimeter (0.008 to 0.012 inch) in a first direction parallel to the surfaces of the backing 26. The stem portions each have a width dimension in the range of 0.018 to 0.03 centimeter (0.007 to 0.012 inch) in a second direction generally at a right angle to the first direction and parallel to the surfaces of the backing 26 (which second direction is aligned with the length of the strip), and the head portions each have a width dimension in the second direction that is between 0.007 and 0.038 centimeter (0.003 and 0.015 inch) greater than the width dimension of the stem portion, less than 0.1 centimeter, and preferably in the range of 0.04 to 0.065 centimeter (0.016 to 0.026 inch). Hook members 25 of this small size have been found to easily penetrate between and engage the loops on loop fastener portions, but individually have little holding power so that the hook fastener portion 24 includes at least 45, and preferably 70 to 100 per square centimeter (at least 300 and preferably 450 to 645 hook members 25 per square inch) of the spaced hook members 25 projecting from the upper surface of the backing 26 to provide the required holding power, while the total cross sectional area occupied by the head portions in a plane parallel to the upper surface is less that 32 percent and preferably in the range of 5 to 15 percent of the area of the upper surface to retain the ease of engagement of the large number of projecting hook members 25 with the loop fastener portion 15.

Preferably the hook fastener portion 24 is made of a polypropylene/polyethylene copolymer or a blend of polypropylene with an ethylene-vinyl acetate block copolymer or a styrene-ethylene-butylene-styrene block copolymer, and has an elastic modulus within the range from 100 to 500 megaPascals as measured in said second direction (i.e., the direction the parts of the head portion project over the stem portion) according to ASTM D 882.80a, which measurement generally comprises measuring the initial slope of the stress strain curve from a tensile test of the material. Hook members 25 with an elastic modulus in that range exhibit an excellent ability to initially engage the loop fastener portion 15, and to resist shear and peel forces tending to separate them from the loop fastener portion 15 once they are engaged. This combination of properties is believed to be due to the ability of the hook members 25 to resiliently bend to move between loops during engagement, and to resiliently bend when they are pulled out of engagement with the loops which resilient bending minimizes breaking of both the small hook members 25 and the loops, and thus prolongs the useful life and esthetics of both the hook fastener portion and the loop fastener portion with which it is mated.

Hook fastener portions having the number of hook portions per unit area of the size and made of the polymeric material indicated above have found to have a very smooth and non abrasive feel when the hook portions are pressed against a persons skin, which is desirable so that the hook portions will not cause discomfort or injury to the skin of a person with which inadvertent contact with the hook portions is made.

The hook fastener portions 24 are made by an adaptation of a known method of making hook fastener portions described in U.S. patents Nos. 3,226,113; 3,557,413; 4,001,366; 4,056,593; and 4,189,098, which method generally includes extruding a thermoplastic resin through a die shaped to form a backing 26 layer and spaced ridges projecting above an upper surface of the backing 26 layer that have the cross sectional shape of the hook members 25 to be formed, transversely cutting the ridges at spaced locations along their length to form discrete portions of the ridges, and stretching the backing 26 layer to separate those portions of the ridges which are then the spaced hook members 25.

Figure 4 schematically illustrates attaching a layered structure 48 including one of the hook fastener portions 24, and the strip 18 and the layer 27 of pressure sensitive adhesive in the form of a length of tape to a concatenation of laminates 11 to be formed into diapers 10 moving along a production line. A strip 36 of hook material made by the method described above from which the hook fastener portion 24 can be cut and pressure sensitive adhesive coated tape 37 from rolls 38 and 39 respectively are joined at a roller 40 with the hook material 36 along one edge of the tape 37, whereupon the composite 41 thus formed is guided and fed by nip rollers 44 onto the periphery of a vacuum wheel 45 having a greater peripheral speed than the speed of the composite 41 is fed by the nipping rollers 44. The end of the composite 41 slips on the periphery of the vacuum wheel 45 until a timed cutter 46 cuts an end portion therefrom which forms the layered structure 48 and is then carried into engagement with one of the laminates 11 moving past the vacuum wheel 45.

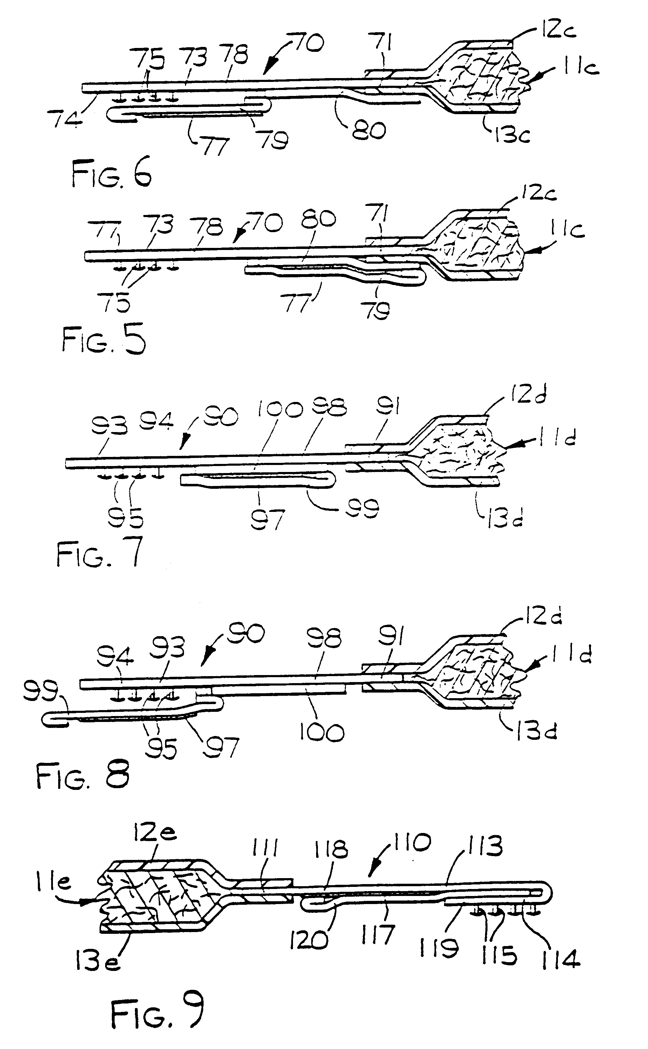

Figures 5 and 6 illustrate a tab assembly 70 of a diaper 10c similar to the diaper 10 illustrated in Figure 1 for which diaper 10c similar parts have been identified with the same reference numerals to which the suffix "c" has been added. Like the tab assembly 20, the tab assembly 70 has a first end portion 71 attached to the laminate 11c and has a distal end portion 73 unattached to the laminate 11c, and a hook fastener means or portion 74 comprising a plurality of projecting hook members 75 along its distal end portion 73 that are adapted to make releasable engagement with the loops on a loop fastener portion. The tab assembly 70 includes an elongate polymeric strip 78 having an end portion bonded (e.g., by adhesive, heat bonding, or sonic sealing) between the film 12c and nonwoven layer 13c of the laminate 11c, and a layer 77 of pressure sensitive adhesive adjacent the projecting hook members 75 on the distal end portion 73. The layer 77 of pressure sensitive adhesive is coated on a backing 79 attached at one end as by sonic welding to the strip and releasably adhered in a ready position of the tab assembly 70 (shown in Figure 5) to a release liner 80 adhered along portions of the strip 78 and nonwoven layer 13c, at which ready position the hook members 75 could be engaged with a loop fastener portion to attach the diaper 10c to an individual. The backing 79 and attached layer 77 of pressure sensitive adhesive can be manually pealed away from the release liner 80 to position the layer 77 of pressure sensitive adhesive on the backing over the hook members 75 in a dispose position as shown in Figure 10 to afford securing the diaper 10c, when soiled and removed, in a rolled or folded condition surrounding the soiled portion of the diaper 10c to facilitate its disposal by engagement of the layer 77 of pressure sensitive adhesive of the backing 79 with the film 12c or other portions of the laminate 11c (not shown).

The hook members 75 of the hook fastener means or portion 74 are illustrated as being integral with the strip 78, however separate hook fastener portions of the type described with reference to Figures 1 through 3 could be adhered to the end of the strip 78.

Figures 7 and 8 illustrate an alternate embodiment of a tab assembly (90) of a diaper 10d similar to the diaper 10 illustrated in Figure 1 for which diaper 10d similar parts have been identified with the same reference numerals to which the suffix "d" has been added. Like the tab assembly 20, the tab assembly 90 has a first end portion 91 attached to the laminate 11c and has a distal end portion 93 unattached to the laminate 11c, and a hook fastener means or portion 94 comprising a plurality of projecting hook members 95 along its distal end portion 93 that are adapted to make releasable engagement with the loops on a loop fastener portion. The tab assembly 90 includes an elongate polymeric strip 98 having an end portion bonded between the film 12d and nonwoven layer 13d of the laminate 11d and a layer 97 of pressure-sensitive adhesive adjacent the projecting hook members on the distal end portion 93. The layer 97 of pressure sensitive adhesive is coated on a backing 99 (i.e., the backing 99 and the layer 97 of pressure sensitive adhesive are a piece of pressure sensitive adhesive coated tape) attached at one end as by sonic welding to the strip 98 and releasably adhered in a ready position of the tab assembly 90 (shown in Figure 7) to a release liner 100 adhered along a central portion of the strip 98, at which ready position the hook members 95 could be engaged with a loop fastener portion to attach the diaper 10d to an individual. The backing 99 and attached layer 97 of pressure sensitive adhesive can be pealed away from the release liner 100 to position the layer of pressure sensitive adhesive over the hook members 95 in a dispose position of the tab assembly 70 as shown in figure 8 to afford securing the diaper 10d when soiled and removed, in a rolled or folded condition surrounding the soiled portion of the diaper 10d to facilitate its disposal by engagement of the layer 97 of pressure sensitive adhesive on the backing 99 with the film 12d or other portions of the laminate 11d (not shown).

Figure 9 illustrates a second alternate embodiment of a tab assembly 110 of a diaper 10e similar to the diaper 10 illustrated in Figure 1 for which diaper 10e similar parts have been identified with the same reference numerals to which the suffix "e" has been added. Like the tab assembly 20, the tab assembly 110 has a first end portion 111 attached to the laminate 11e and has a distal end portion 113 unattached to the laminate 11e, and a hook fastener means or portion 114 comprising a plurality of projecting hook members 115 along its distal end portion 113 that are adapted to make releasable engagement with the loops on a loop fastener portion. The tab assembly 110 includes an elongate polymeric strip 118 having an end portion bonded between the film 12e and nonwoven layer 13e of the laminate 11e and a layer 117 of pressure-sensitive adhesive coated on or adhered to the strip 118 adjacent the projecting hook members on the distal end portion 113. An end part 119 of the portion of the polymeric strip 118 defining the distal end portion 113 and carrying the hook fastener portion 114 is folded over and fixed to an end portion of a release liner 120 releasably adhered over the layer 117 of pressure sensitive adhesive. The hook fastener portion 113 is engagable with the loop fastener portion with the end part 119 folded over in a ready position as shown in Figure 9 , and the portion of the release liner 120 adhered to the layer 117 of pressure sensitive adhesive is separable from the layer 117 of pressure sensitive adhesive to expose it in a dispose position of the tab assembly 110 (not shown) to afford securing the diaper 10e, when soiled and removed, in a rolled or folded condition surrounding the soiled portion of the diaper 10e to facilitate its disposal by engagement of the layer 117 of pressure sensitive adhesive with the film 12e or other portion of the laminate 11e (not shown).

## Claims

1. A disposable garment or diaper including a laminate (11c, 11d) having first and second opposite ends, and hook and loop fastener means for fastening together portions of said laminate (11c, 11d) to secure said diaper to an individual, said fastener means including loop fastener portion means adjacent the first end of said laminate comprising a multiplicity of loops, a pair of flexible elongate polymeric tab assemblies (70, 90) including elongate polymeric strips (78, 98) having first end portions (71, 91) attached to said laminate (11c, 11d) adjacent the second end of said laminate (11c, 11d) and having distal end portions (73, 93) unattached to said laminate (11c, 11d), and hook fastener portion means (74, 94) at the distal end portions of said strips (78, 98) comprising a plurality of projecting hook members (75, 95) adapted to make releasable engagement with said loops, said tab assemblies (70, 90) including a layer (77, 97) of pressure sensitive adhesive and a release liner (80, 100) releasably adhered over the layer (77, 97) of pressure-sensitive adhesive providing, after the diaper has been soiled and removed from the individual, means for securing the soiled diaper in a rolled or folded condition surrounding the soiled portion of the diaper to facilitate its disposal by engagement of the layer (77, 97) of pressure sensitive adhesive with said laminate (11c, 11d), said disposable garment or diaper being characterized by the feature that the release liner (80, 100) is adhered along portions of the strip (78, 98) and the layer (77, 97) of pressure sensitive adhesive is coated on a backing (79, 99) attached at one end to the strip (78, 98) and releasably adhered in a ready position of the tab assembly (70, 90) to the release liner (80, 100), at which ready position the hook members (75, 95) can be engaged with the loop fastener portion to attach the diaper to an individual, said backing (79, 99) and attached layer (77, 97) of pressure sensitive adhesive being manually pealable away from the release liner (80, 100) to position the layer (77, 97) of pressure sensitive adhesive on the backing (79, 99) over the hook members (75, 95) in a dispose position to afford securing the diaper when soiled and removed, in a rolled or folded condition surrounding the soiled portion of the diaper to facilitate its disposal by engagement of the layer (77, 97) of pressure sensitive adhesive on the backing (79, 99) with the laminate (11c, 11d).

2. A disposable garment or diaper including a laminate (11e) having first and second opposite ends, and hook and loop fastener means for fastening together portions of said laminate (11e) to secure said diaper to an individual, said fastener means including loop fastener portion means adjacent the first end of said laminate comprising a multiplicity of loops, a pair of flexible elongate polymeric tab assemblies (110) including elongate polymeric strips (118) having first end portions (111) attached to said laminate (11e) adjacent the second end of said laminate (11e) and having distal end portions (113) unattached to said laminate (11e), and hook fastener portion means (114) at the distal end portions of said strips (118) comprising a plurality of projecting hook members (115) adapted to make releasable engagement with said loops, said tab assemblies (110) including a layer (117) of pressure sensitive adhesive and a release liner (120) releasably adhered over the layer (117) of pressure-sensitive adhesive providing, after the diaper has been soiled and removed from the individual, means for securing the soiled diaper in a rolled or folded condition surrounding the soiled portion of the diaper to facilitate its disposal by engagement of the layer (117) of pressure sensitive adhesive with said laminate (11e), said disposable garment or diaper being characterized by the feature that an end part (119) of the portion of the polymeric strip (118) defining the distal end portion (113) and carrying the hook fastener portion (114) is folded over and fixed to an end portion of the release liner (120) releasably adhered over the layer (117) of pressure sensitive adhesive.

## Patentansprüche

1. Wegwerfkleidungsstück oder -windel umfassend einen Schichtpreßstoff (11c, 11d) mit ersten und zweiten einander gegenüberliegenden Enden, und einen Haftverschluß, der die Abschnitte des Schichtpreßstoffs (11c, 11d) miteinander verbindet, um die Windel an einer Person zu befestigen, wobei der Haftverschluß einen mit Schlingen versehenen Befestigungsabschnitt im Bereich des ersten Endes des Schichtpreßstoffes aufweist, der aus einer Vielzahl von Schlingen besteht, ein Paar flexibler, langgestreckter Laschenanordnungen (70, 90) aus einem Polymer umfassend langgestreckte Polymerstreifen (78, 98) mit ersten Endabschnitten (71, 91), die an dem Schichtpreßstoff (11c, 11d) im Bereich des zweiten Endes des Schichtpreßstoffes (11c, 11d) befestigt sind, und mit distalen Endabschnitten (73, 93), die nicht an dem Schichtpreßstoff (11c, 11d) befestigt sind, und einen mit Haken versehenen Befestigungsabschnitt (74, 94) an den distalen Endabschnitten der Streifen (78, 98), der eine Vielzahl von hervorstehenden Hakenelementen (75, 95) umfaßt, die mit den Schlingen in lösbaren Eingriff treten können, wobei die Laschenanordnungen (70, 90) eine Haftkleberschicht (77, 97) und eine Trennfolie (80, 100) aufweisen, die lösbar auf die Haftkleberschicht (77, 97) aufgebracht ist, so daß dann, wenn die Windel verschmutzt ist und der Person abgenommen wurde, ein Mittel vorhanden ist, um die verschmutzte Windel in einem um den verschmutzten Abschnitt der Windel herum zusammgerollten oder -gefalteten Zustand zu sichern, damit sie leichter entsorgt werden kann, indem die Haftkleberschicht (77, 97) mit dem Schichtpreßstoff (11c, 11d) verbunden wird, wobei das Wegwerfkleidungsstück bzw. die Wegwerfwindel dadurch gekennzeichnet ist, daß die Trennfolie (80, 100) entlang von Abschnitten des Streifens (78, 98) aufgebracht ist, und die Haftkleberschicht (77, 97) auf einen an einem Ende an dem Streifen (78, 98) befestigten Träger (79, 99) aufgetragen ist und in einer Bereitschaftsstellung der Laschenanordnung (70, 90) lösbar mit der Trennfolie (80, 100) verbunden ist, wobei die Hakenelemente (75, 95) in der Bereitschaftsstellung mit dem mit Schlingen versehenen Befestigungsabschnitt in Eingriff treten können, um die Windel an einer Person zu befestigen, wobei der Träger (79, 99) und die aufgetragene Haftkleberschicht (77, 97) von Hand von der Trennfolie (80, 100) abgezogen werden können, um die Haftkleberschicht (77, 97) auf dem Träger (79, 99) über den Hakenelementen (75, 95) in einer Wegwerfposition zu positionieren, damit die Windel dann, wenn sie verschmutzt ist und abgenommen wurde, in einem den verschmutzten Abschnitt umgebenden zusammengerollten oder -gefalteten Zustand gesichert werden kann, so daß sie leichter entsorgt werden kann, indem die Haftkleberschicht (77, 97) auf dem Träger (79, 99) mit dem Schichtpreßstoff (11c, 11d) verbunden wird.

2. Wegwerfkleidungsstück oder -windel umfassend einen Schichtpreßstoff (11e) mit ersten und zweiten einander gegenüberliegenden Enden, und einen Haftverschluß, der die Abschnitte des Schichtpreßstoffs (11e) miteinander verbindet, um die Windel an einer Person zu befestigen, wobei der Haftverschluß einen mit Schlingen versehenen Befestigungsabschnitt im Bereich des ersten Endes des Schichtpreßstoffes aufweist, der aus einer Vielzahl von Schlingen besteht, ein Paar flexibler, langgestreckter Laschenanordnungen (110) aus einem Polymer umfassend langgestreckte Polymerstreifen (118) mit ersten Endabschnitten (111), die an dem Schichtpreßstoff (11e) im Bereich des zweiten Endes des Schichtpreßstoffes (11e) befestigt sind, und mit distalen Endabschnitten (113), die nicht an dem Schichtpreßstoff (11e) befestigt sind, und einen mit Haken versehenen Befestigungsabschnitt (114) an den distalen Endabschnitten der Streifen (118), der eine Vielzahl von hervorstehenden Hakenelementen (115) umfaßt, die mit den Schlingen in lösbaren Eingriff treten können, wobei die Laschenanordnungen (110) eine Haftkleberschicht (117) und eine Trennfolie (120) aufweisen, die lösbar auf die Haftkleberschicht (117) aufgebracht ist, so daß dann, wenn die Windel verschmutzt ist und der Person abgenommen wurde, ein Mittel vorhanden ist, um die verschmutzte Windel in einem um den verschmutzten Abschnitt der Windel herum zusammgerollten oder -gefalteten Zustand zu sichern, damit sie leichter entsorgt werden kann, indem die Haftkleberschicht (117) mit dem Schichtpreßstoff (11e) verbunden wird, wobei das Wegwerfkleidungsstück bzw. die Wegwerfwindel dadurch gekennzeichnet ist, daß ein Endteil (119) des Abschnitts des Polymerstreifens (118), der den distalen Endabschnitt (113) bildet und den mit Haken versehenen Befestigungsabschnitt (114) trägt, umgeklappt und an einem Endabschnitt der Trennfolie (120) befestigt wird, die lösbar auf die Haftkleberschicht (117) aufgebracht ist.

## Revendications

1. Sous-vêtement ou couche absorbante jetable comprenant un stratifié (11c,11d) qui présente une première et une deuxième extrémités opposées, et des moyens d'attache à crochets et boucles pour relier l'une à l'autre des parties dudit stratifié (11c,11d) afin de fixer ladite couche à une personne, lesdits moyens d'attache incluant une partie d'attache à boucles placée près de la première extrémité dudit stratifié et comportant une multiplicité de boucles, deux pattes allongées flexibles en polymère (70,90) incluant des bandes depolymère allongées (78,98) qui présentent des premières parties d'extrémité (71,91) fixées audit stratifié (11c,11d) près de la deuxième extrémité dudit stratifié (11c,11d) et des parties d'extrémité libres (73, 93) non fixées audit stratifié (11c,11d), et des parties d'attache à crochets (74,94) prévues dans les parties d'extrémité libres desdites bandes (78,98) et comportant une pluralité de crochets en saillie (75,95) qui peuvent venir en prise libérable avec lesdites boucles, lesdites pattes (70,90) incluant une couche (77,97) d'adhésif sensible à la pression et une pellicule anti-adhérence (80, 100) collée de façon détachable sur la couche (77,97) d'adhésif sensible à la pression qui constitue, lorsque la couche a été salie et enlevée de la personne, un moyen d'attache de la couche salie dans un état roulé ou plié autour de la partie salie de la couche afin de faciliter son rejet, par collage de la couche (77, 97) d'adhésif sensible à la pression avec ledit stratifié (11c,11d), ledit sous-vêtement ou couche jetable étant caractérisé en ce que la pellicule anti-adhérence (80,100) est collée le long de parties de la bande (78, 98) et la couche (77,97) d'adhésif sensible à la pression est appliquée sur un support (79,99) fixé, à une extrémité, à la bande (78,98) et collé de façon détachable, dans une position prête à l'emploi de la patte (70,90), à la pellicule anti-adhérence (80,100), les crochets (75,95) pouvant venir en prise dans ladite position prête à l'emploi avec la partie d'attache à boucles pour fixer la couche à une personne, ledit support (79, 99) et la couche associée (77,97) d'adhésif sensible à la pression pouvant être décollés manuellement de la pellicule anti-adhérence (80,100) de manière à positionner la couche (77,97) d'adhésif sensible à la pression prévue sur le support (79,99) au-dessus des crochets (75,95), dans une position de rejet, pour permettre d'attacher la couche, lorsqu'elle est salie et enlevée, dans un état roulé ou plié autour de la partie salie de la couche, afin de faciliter son évacuation, par collage de la couche (77,97) d'adhésif sensible à la pression prévue sur le support (79,99) au stratifié (11c, 11d).

2. Sous-vêtement ou couche jetable comprenant unstratifié (11e) qui présente une première et une deuxième extrémités opposées, et des moyens d'attache à crochets et boucles pour relier l'une à l'autre des parties dudit stratifié (11e) afin de fixer ladite couche à une personne, lesdits moyens d'attache incluant une partie d'attache à boucles proche de la première extrémité dudit stratifié et comportant une multiplicité de boucles, deux pattes allongées flexibles en polymère (110) incluant des bandes allongées de polymère (118) qui présentent des premières parties d'extrémité (111),fixées au dit stratifié (11e) près de la deuxième extrémité du dit stratifié (11e), et des parties d'extrémité libres 113 non fixées audit stratifié (11e), et des parties d'attache à crochets (114) prévues dans les parties d'extrémité libres desdites bandes (118) et comportant une pluralité de crochets en saillie (115) qui peuvent venir en prise libérable avec lesdites boucles, lesdites pattes (110) incluant une couche (117) d'adhésif sensible à la pression et une pellicule anti-adhérence (120) collée de façon séparable sur la couche (117) d'adhésif sensible à la pression qui constitue, lorsque la couche a été salie et enlevée de la personne,un moyen d'attache de la couche salie dans un état roulé ou plié autour de la partie salie de la couche afin de faciliter son rejet, par collage de la couche (117) d'adhésif sensible à la pression audit stratifié (11e), ledit sous-vêtement ou couche jetable étant caractérisé en ce qu'une partie d'extrémité (119) de la région de la bande de polymère (118) définissant la partie d'extrémité libre (113) et portant la partie d'attache à crochets (114) est rabattue sur une partie d'extrémité de la pellicule anti-adhérence (120) et fixée à celle-ci, ladite pellicule anti-adhérence étant collée de façon détachable sur la couche (117) d'adhésif sensible à la pression.
